# EUROPEAN PATENT SPECIFICATION

(11) **EP 1 971 449 B1**
(45) Date of publication and mention of the grant of the patent: **09.03.2016**
(21) Application number: 06829881.9
(22) Date of filing: 27.12.2006
(51) Int. Cl.: B09B 3/00, A61L 11/00, C05F 9/02

(54) **METHOD AND DEVICE DESIGNATED FOR THE TREATMENT OF WASTE**
ZUR BEHANDLUNG VON ABFALL BESTIMMTE(S) VERFAHREN UND VORRICHTUNG
PROCEDE ET DISPOSITIF PREVUS POUR LE TRAITEMENT DE DECHETS

(30) Priority: 10.01.2006 ES 200600046
(43) Date of publication of application: 24.09.2008
(73) Proprietor: Ambiensys, S.L., 08213 Polinya (ES)
(72) Inventor: RIBAS LOPEZ, Rafael, E-08213 Polinya (ES); RIBAS MARTINEZ, Oscar, E-08213 Polinya (ES)
(74) Representative: Sugrañes Patentes y Marcas
(86) International application number: PCT/EP2006/012556
(87) International publication number: WO 2007/079968

(56) References cited:
- WO-A-82/01483
- WO-A-03/025101
- US-A- 5 119 994
- US-A- 5 427 650
- US-A- 5 556 445
- US-A- 6 050 423

## Description

### Technical field of the invention

The invention refers to a waste treatment method, particularly for the treatment of urban solid waste, wherein the waste to be treated is subject to overpressure and overtemperature conditions for a given period, sufficient for the sterilisation and transformation thereof into useable subproducts, as well as a device to perform said method.

### Background of the invention

Currently, there are different methods designed for the treatment of urban solid waste or household refuse, as well as equipment that is especially adapted so as to perform said methods, the purpose whereof is making the waste suitable to facilitate the subsequent separation and recovery thereof by means of recycling and/or energy utilisation circuits.

In the field of the art, a well-known practise is the so-called waste sanitation, wherein the waste is sterilised and partially desiccated, and wherein the organic fraction of the waste is converted into an energetically useable homogenised subproduct. To this end, the waste is exposed to a water vapour bath, at high temperature, for a predetermined period of time, while being mechanically agitated with the purpose, amongst others, of extracting the liquids contained in the waste by means of an especially adapted drainage and extraction system. Normally, the waste treatment is performed in a hermetically sealed container, such as a pressure tank or autoclave, wherein the waste is agitated and subject to the vapour bath.

Unlike autoclaves used to sterilise food, reuseable medical utensils or containers, wherein the objects to be sterilised must be immobilised, in autoclaves designed for waste treatment it is essential, as explained above, that the waste introduced into the autoclave be mechanically mixed and agitated inside, which, in addition to causing the release of the liquids contained therein, ensures contact of all the waste with the water vapour inside the autoclave, as well as the formation of organic fibre conglomerates as energetically useable subproducts.

On the other hand, the above-mentioned autoclaves are only adapted to drain the condensed vapour, unlike the autoclaves used for the treatment of urban solid waste, wherein it is necessary to extract from the interior of the autoclave the liquids released by the waste, some of which require a subsequent cleaning treatment, in addition to the vapour condensed inside the autoclave.

Another big difference is that the great amount of waste generated in large population centres makes it necessary to increase the dimensions of the autoclaves designed for waste treatment, since the treatment facilities must have sufficient capacity to simultaneously treat a high volume of waste.

For all these reasons, the configuration and technical characteristics of the autoclaves and similar devices designed to treat urban solid waste are very different from those autoclaves used in the food or health sectors.

In the first place, in order to subject the waste to constant agitation during treatment in the interior of the autoclaves, the latter are conventionally formed by large, rotating, cylindrical containers or pressure tanks, which are capable of rotating around their longitudinal axis. This solution entails the use of suitable materials capable of resisting the mechanical stresses and oscillations whereto the containers are subject due to their rotational character, and, simultaneously, of resisting the high pressures and temperatures in the interior of the containers. Similarly, the containers' interior walls must resist the mechanical aggressions whereto they are subject as a result of the fact that, upon being agitated, the solid waste constantly hits and rubs the containers' interior walls, as well as the chemical aggressions caused by the liquids released from the solid waste during the treatment thereof, some of which are corrosive.

As examples of containers such as the ones described above, we cite documents US 5427650 and WO 03025101, which are representative of the state of the art.

Document US 5427650 describes a waste treatment method and a container or pressure tank designed to perform said method. This container is cylindrical and rotating, and is equipped, on one end, with an opening designed for the introduction and extraction of the waste to be treated, and, on the opposite end, with a passageway for the introduction of water vapour into the container. The vapour condensed in the interior of the container is extracted through the same passageway wherethrough the vapour is introduced and is suctioned when the interior of the container is subject to overpressure.

Document WO 03025101 describes a waste treatment method in a hermetically sealed container, wherein the waste material is treated with a vapour source at a temperature of at least 130ºC and a pressure above 2 barg (measured bar), for at least 20 minutes, whilst subjecting the container to rotation and mixing the waste material by means of helicoidal blades located, to this end, on the container's interior walls. Said container is equipped, on one end, with an opening designed for the introduction and extraction of the waste, and, on the opposite end, with a passageway for the entry of the water vapour. Unlike the container described in document US 5427650, this rotating container is equipped with an independent passageway for the exit of the condensates and the liquids released by the waste, also located on the end opposite the end equipped with the opening for the introduction and extraction of the waste. The container's longitudinal axis is slightly tilted with respect to the horizontal whilst the waste is being treated in the interior; for this reason, the liquids released during treatment are accumulated in the area of the container equipped with the passageway designed for exit of the liquids. In order to prevent plugging of the extraction passageway, the container is internally equipped with a filtering device which allows for passage of the liquids towards the exit passageway, but prevents the passage of waste.

In addition to the above-mentioned disadvantages, related to the mechanical stresses and the internal wear whereto these rotating containers are subject, the containers described in the cited documents exhibit other disadvantages. In the first place, rotation of the container around its axis complicates communications between the interior and the exterior of the container, particularly in relation to the passageways designed for entry of the water vapour and exit of the liquids, if one desires to prevent altering the pressure and temperature conditions in the interior of the container and hindering the rotational movement whereto the latter is subject. For this purpose, in the embodiments described in documents US 5427650 and WO 03025101, the containers are equipped, on the closed end, with rotating sealed joints located on the longitudinal axis which allow the conduits that communicate the container with the exterior to remain fixed whilst the container rotates around the axis. This solution makes it possible to extract the liquids and condensates from the interior of the container without interrupting the rotation of the latter, but greatly increases the cost of manufacturing the containers, introduces components which require high maintenance, such as the rotating sealed joint, and limits the location,of said passageways to a very specific area of the containers' closed end, thereby preventing, for example, good dispersion of the vapour introduced into the containers.

Document US 5119994 describes an apparatus for processing medical waste materials comprising a fixed elongate pressure vessel housing an elongate drum of generally cylindrical configuration for rotation about its longitudinal axis.

Document US 5556445 describes a process for treating municipal waste without use of a pressure vessel, using an apparatus in which organic fraction of the waste material pass through the perforations of a perforated drum.

Document WO 8201483 discloses a prior art waste treatment method and a device for the treatment of waste in accordance with the preamble of claim 6. This document describes a process wherein a perforated drum is used in this case to force the organic components of the waste material to flow through during a sudden depressurization operation.

In the second place, the extraction of liquids from the interior of the containers is not very efficient and, therefore, the waste contains a high level of humidity once treatment in the interior of the containers is finished. Furthermore, another disadvantage lies in the filters which are necessary to allow for passage of the liquids and condensates and to retain the solid waste, since these filters usually require high maintenance and it is common to have to replace them with new filters in short periods of time, which entails interrupting the operation of the facilities.

For these reasons, it would be desirable to have available a waste treatment method, particularly for the treatment of urban solid waste, which might improve the extraction of liquids from inside the treated waste, improving the drying of said waste, as well as a device designed to perform said method, of the type comprising a sealed container or pressure tank, which makes it possible to significantly reduce the current manufacturing costs and maintenance costs of these types of devices, and allows to improve the distribution of water vapour in the interior of the container, as well as the extraction of liquids from the interior during treatment of the waste.

### Description of the invention

The waste treatment method, which is the first object of this invention, is in accordance with claim 1 and comprises the following steps: introducing the waste to be treated inside a tubular body the lateral walls whereof are equipped with multiple perforations; and subjecting the assembly formed by the tubular body and the waste introduced therein to a gas working environment at a pressure above 4 bar and a temperature between 115ºC and 200ºC, for at least 15 minutes, and simultaneously rotating the tubular body around its longitudinal axis in order to mechanically agitate the introduced waste, the perforations of the lateral walls being dimensioned to prevent the waste flowing towards the exterior of the tubular body but allowing the liquids released from the waste flowing to the exterior of the tubular body through the perforations in the lateral walls. In order to subject the assembly formed by the tubular body and the waste introduced therein to the above-mentioned working environment, the method comprises, subsequent to the introduction of the waste into said tubular body, enclosing the tubular body in a sealed working enclosure and injecting gas at a temperature between 115ºC and 200ºC into the container until a pressure above 4 bar is reached.

The method further comprises, after subjecting the assembly formed by the tubular body and the waste introduced therein to the above-mentioned working environment, the step of gradually reducing the pressure of the gas working environment until the initial atmospheric pressure is reached, by extracting said gas from the working enclosure and, simultaneously, cooling and drying the waste introduced into the tubular body by injecting a drying gas into the working enclosure, which reaches the waste through the perforations in the lateral walls of said tubular body.

Preferably, the gas injected into the working enclosure is directed against the tubular body by diffuser devices placed facing one another and regularly distributed along the length of the tubular body.

According to one embodiment, the injected gas is water vapour.

Preferably, the drying gas injected into the working enclosure is directed against the tubular body by diffuser devices placed facing one another and regularly distributed along the length of the tubular body.

According to one embodiment, the injected drying gas is dry air.

According to a preferred characteristic of the method, during the simultaneous operations of reducing the pressure of the gas working environment and cooling and drying the waste, the tubular body rotates around its longitudinal axis.

According to another preferred characteristic of the method, the liquids which flow to the exterior of the tubular body through the perforations in the lateral walls thereof and the water vapour condensed in the interior of the working enclosure are collected by the back wall of the above-mentioned working enclosure, and the method comprises extracting said collected liquids from the working enclosure in a controlled manner, thereby reducing the level of humidity inside said working enclosure and, as a result, the level of humidity of the waste.

The waste treatment device, particularly designed for the treatment of urban solid waste, which is also an object of this invention, is in accordance with claim 6 and comprises a pressure tank equipped with an inlet opening for the introduction and extraction of waste into and out of the tank, at least one inlet for a gas, such as water vapour or air, and at least one outlet for the condensates, the liquids released by the waste during treatment and the gas itself.

In essence, the pressure tank is fixed and the device additionally comprises a rotating cylindrical tubular body, open on one end and closed on the opposite end by means of a lid, which is placed in the interior of the pressure tank and is designed to contain the waste introduced into the tank and to mechanically agitate it upon rotating the tubular body around its longitudinal axis; the lateral walls of the tubular body are equipped with multiple perforations wherethrough the gas may penetrate into the tubular body and the liquids may flow towards the exterior thereof, being collected by the pressure tank.

These characteristics prevent direct contact of the waste with the inner surfaces of the tank walls and the need to apply a rotational movement to the above-mentioned pressure tank, which is a large, heavy structure.

According to a preferred characteristic of the invention, the pressure tank comprises a main cylindrical tubular portion, closed on the rear end by an outer lid, and the tubular body is placed coaxially with respect to said main portion and, through the use of sliding means, leans against the inner surfaces of the pressure tank walls, an intermediate chamber being formed between the main portion of the pressure tank and the tubular body,

According to another preferred characteristic of the invention, the lid of the tubular body is placed facing the outer lid of the main portion of the pressure tank and is joined to a driving axis which crosses the above-mentioned outer lid of the main portion of the pressure tank through a sealed joint and is coupled to an engine located on the exterior of said pressure tank.

Another preferred aspect worth highlighting in the device described in the invention is that the pressure tank comprises a hollow, tubular front portion, with an increasing cross-section, joined without interruption to the open end of the main portion on the end with the largest cross-section, the inlet opening of the pressure tank being located on the end with the smallest cross-section, with a detachable gate that may be hermetically coupled to the above-mentioned opening.

According to another preferred characteristic of the device of the invention, the tubular body practically covers the entire main portion of the pressure tank, and the open end of the above-mentioned tubular body protrudes from the snout of the inlet opening of said pressure tank.

Another preferred characteristic of the device of the invention consists of the fact that the tubular body is internally equipped with a series of longitudinal blades and/or helicoidal blades that protrude from the inner surface of the lateral wall of the above-mentioned tubular body, which are designed to stir and to longitudinally displace, respectively, the waste introduced into said tubular body upon rotation, in one direction or another, of the tubular body around its longitudinal axis.

According to one embodiment, the gas inlets are placed on the upper part of the lateral wall of the main cylindrical portion of the pressure tank.

According to another preferred characteristic, the gas inlets are connected to at least one conduit which extends longitudinally in the interior of the pressure tank, along the intermediate chamber, said conduits being equipped with a series of diffuser devices wherethrough the gas is expelled inside the pressure tank.

According to a variant of the invention, the main cylindrical portion of the pressure tank is tilted with respect to the horizontal, in order to allow for the channelling and conduction of the liquid collected by the pressure tank towards the lowermost part of the above-mentioned pressure tank, and the outlet thereof is placed on this lowermost part, being equipped with a valve element for the controlled extraction of the liquids accumulated in said lowermost part to the exterior of the pressure tank.

According to a particularly interesting embodiment, the tubular body comprises a frame covered by plates that are joined to one another through joining means, said frame being preferably composed of rings that are transversely placed with respect to its longitudinal axis and separated by a certain distance, joined to one another by means of longitudinal rods.

According to another embodiment, the pressure tank is made of carbon steel and the pressure tank walls comprise, on the inner face, a primer layer designed to protect them against the corrosive elements which the waste introduced therein may contain, as well as against the constant humid environment whereto they will be exposed, of the aluzinc type or similar.

According to another embodiment, the pressure tank walls comprise, on the outer face, a thermal insulation coating, which preferably comprises an inner layer made of thermal insulation material, such as rock wool or similar, and an embellishing outer layer made of stainless steel.

According to another embodiment, the inlet opening of the pressure tank comprises support means which are adapted so as to receive, at least partially, the contents of a conveyor belt capable of being introduced through the same opening in order to pour the waste into the pressure tank.

### Brief description of the drawings

The sheet of drawings included herein represents an embodiment example, not limited thereto, of the waste treatment device which is the object of this invention. In said drawings:
- Fig.1: is a perspective side view of the waste treatment device;
- Fig. 2: is another perspective view, partially sectioned, of the waste treatment device in Fig. 1; and
- Fig. 3: is a schematic cross-section view of the waste treatment device in Fig. 1.

### Detailed description of the drawings

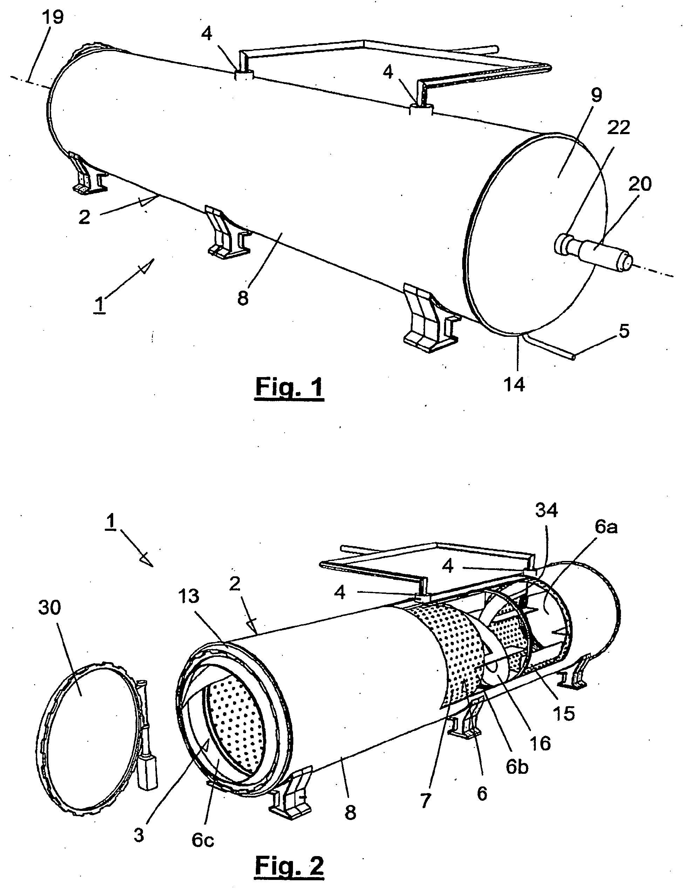

Device 1 represented in Figs. 1 to 3 is designed for the treatment of waste 17 and, more specifically, for the treatment of urban solid waste. Said device 1 comprises a pressure tank 2, which acts as a sealed working enclosure, equipped on one end with an opening 3 that is adapted for the introduction and extraction of waste 17 from the interior, and a gate 30 that is coupled, in a detachable and sealed manner, to said opening 3. In addition, device 1 is equipped with two inlets 4 for a gas, such as water vapour 23 at high temperature or air, preferably dry, depending on the needs at each time, which in turn depend on the stage of the process being performed, as well as an outlet 5 for the condensates, the liquids 25 released by waste 17 during the treatment thereof and the same gas introduced through the above-mentioned inlets 4. Evidently, depending on the length of pressure tank 2, the number of inlets 4 and the distribution thereof may vary, as explained more thoroughly further below.

Fig. 1 shows that pressure tank 2 comprises, in turn, a long main portion 8, which is tubular and cylindrical, one of the ends whereof is closed by an outer lid 9.

According to the represented variant, like the pressure tanks described in the state of the art, longitudinal axis 19 of pressure tank 2 is preferably placed slightly tilted with respect to the horizontal, by approximately 1º. However, unlike said well-known pressure tanks, pressure tank 2 of the invention is designed to remain fixed, joined to the floor whereon it rests, whilst the waste is being treated. Consequently, pressure tank 2 of the invention is not especially adapted to bear the mechanical stresses which are produced, for example, upon rotating the tank around its longitudinal axis 19. For this reason, the material used in its construction and, in particular, the material that makes up the tank's inner surface, is different from the materials used in the construction of the already-known rotating tanks, and the thickness of the walls of tank 2 may even be smaller than that of the rotating tanks described in the state of the art.

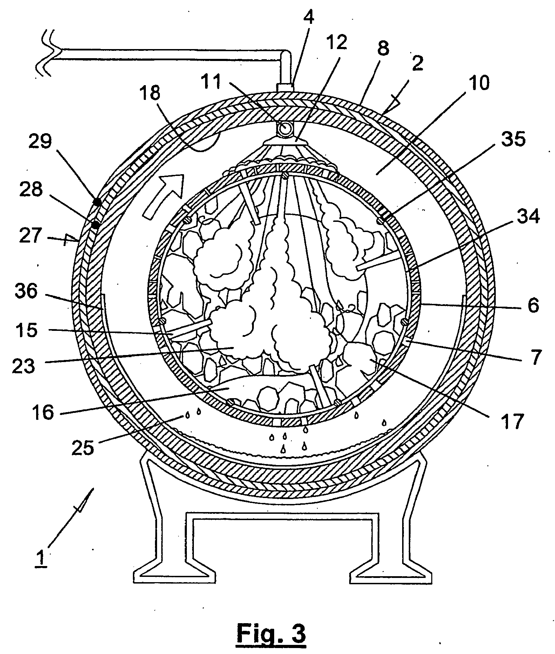

Device 1 comprises, as shown in Figs. 2 and 3, a rotating, cylindrical tubular body 6, designed to contain the waste introduced into pressure tank 2, which is placed in the interior of said pressure tank 2 coaxially with respect to its main portion 8. Said tubular body 6 is open on front end 6c and is closed on the opposite, rear end by means of a lid 6a. In said Figs. 2 and 3, one may also observe that the lateral walls 6b of the above-mentioned tubular body 6 are equipped with multiple perforations 7.

In the sectioned representation shown in Fig. 2, one may observe that tubular body 6 practically covers the entire main portion 8 of pressure tank 2, and that open end 6c of the above-mentioned tubular body 6 protrudes from the snout of opening 3 of said pressure tank 2, thus facilitating the introduction of the waste to be treated therein and preventing the falling of waste particles into the cavity located between the tubular body and the tank during the subsequent extraction of said waste.

By using sliding means, which are not represented, tubular body 6 leans against inner surface 18 of the walls of main portion 8 of pressure tank 2. In a preferred embodiment, said sliding means consist of roller elements located on the inner surface of the lateral wall of main portion 8 of pressure tank 2, against which the above-mentioned tubular body 6 leans. This makes it possible for tubular body 6 to be mounted in the interior of pressure tank 2 in such a way that it may rotate around its longitudinal axis and is incapable of moving in the above-mentioned longitudinal direction, with an intermediate chamber 10 being formed between the inner surface of the lateral wall of main portion 8 of pressure tank 2 and lateral wall 6b of tubular body 6 (see Fig. 3).

It is expected that tubular body 6 may be driven from the exterior by means of a driving axis 20, which transmits a rotational movement, in one direction or another, as desired, to tubular body 6. To this end, lid 6a of tubular body 6, which is placed facing outer lid 9 of main portion 8 of pressure tank 2, is joined to the above-mentioned driving axis 20, which crosses the above-mentioned outer lid 9 of main portion 8 of pressure tank 2 through a sealed joint 22 (see Fig. 1). Although it is not represented, it is understood that driving axis 20 is designed to be coupled to an engine located on the exterior of said pressure tank 2, which transmits the rotational movement to the above-mentioned driving axis 20.

As mentioned above, tubular body 6 is designed to contain all the waste introduced into pressure tank 2. In order to prevent the waste introduced into pressure tank 2 through its opening 3 from becoming lodged in an undesirable manner in intermediate chamber 10, the distance separating pressure tank 2 from tubular body 6 in the area of opening 3 is significantly reduced. For this purpose, the open end of main portion 8 is joined without interruption to a hollow tubular front portion 13, which has a decreasing cross-section in the direction of the snout of open end 6c of the tubular body, with opening 3 of pressure tank 2 being located on the end with the smallest cross-section. Therefore, in the area of the above-mentioned opening 3, the cross-section of pressure tank 2 is fitted to the cross-section of tubular body 6 in such a way that it prevents the introduction of waste between them but, at the same time, allows for relative movement between them, specifically, the rotational movement of tubular body 6.

Figs. 2 and 3 also show that tubular body 6 is internally equipped with a series of longitudinal blades 15 and helicoidal blades 16 which protrude from the inner surface of lateral wall 6b of the above-mentioned tabular body 6. Said blades 15 and 16 are designed to stir and to longitudinally displace, respectively, the waste 17 that has been introduced into and is contained in said tubular body 6, when the latter rotates around its axis during treatment of the waste. In a well-known manner, in order to facilitate the introduction of waste 17 into device 1 and, specifically, into tubular body 6, the above-mentioned tubular body 6 is made to rotate in the forward direction of helicoidal blades 16, which push waste 17 towards the end located opposite opening 3, whilst waste 17 is deposited in the area close to the snout of open end 6c of tubular body 6. Similarly, in order to facilitate the discharge of the waste once the treatment is finished, tubular body 6 is made to rotate, now in the opposite direction, causing helicoidal blades 16 to push waste 17 towards opening 3 of pressure tank 2.

Pressure tank 2 is equipped with two inlets 4 designed to allow for the introduction of water vapour or air under pressure into the tank. Said inlets 4 are placed equidistant from the ends of main portion 8 of pressure tank 2, on the upper part of said main portion. In turn, outlet 5 for the liquids and the gas introduced into pressure tank 2 is placed on the lowermost part 14 of the closed end of main portion 8, close to outer lid 9.

As regards intermediate chamber 10, it is shown in Fig. 3, which schematically represents a cross-section of device 1 of the invention. In said Fig. 3, in order to better show the construction details of device 1, the separation distance between lateral wall 6b of tubular body 6 and the inner surface of the lateral wall of pressure tank 2, which determines the width of intermediate chamber 10, is shown with exaggerated dimensions.

Said Fig. 3 also shows that a conduit 11 extends longitudinally along said intermediate chamber 10, which is connected to inlets 4 for gas, vapour and/or air, depending on the needs, and equipped with a series of diffuser devices 12 wherethrough the gas introduced into pressure tank 2 is expelled. The gas expelled by diffuser devices 12, which are regularly distributed along the length of main portion 8 of pressure tank 2, is directed against the outer face of lateral wall 6b of tubular body 6, reaching the waste 17 that is deposited therein through the perforations 7 provided in the above-mentioned lateral wall 6b of the tubular body.

The above-mentioned perforations 7 also allow for the liquids 25 released by waste 17, which are contained in tubular body 6, to flow towards the exterior of the above-mentioned tubular body 6, being collected and driven through pressure tank 2 towards the outlet 5 that is located on the lowermost part of pressure tank 2.

The walls of pressure tank 2 comprise, on inner face 18, particularly on the lowermost half of main portion 8 of pressure tank 2, a primer layer 36, preferably of the aluzinc type or similar, designed to protect them against the corrosive elements which the waste 17 introduced therein may contain, as well as against the constant humid environment whereto they will be exposed.

According to a particularly interesting construction variant, tubular body 6 comprises a supporting frame, made up of a series of rings 34, placed transversely with respect to longitudinal axis 19 of the tubular body and separated by a certain distance, joined to one another by longitudinal rods 35, which is covered by a series of perforated plates, preferably made of stainless steel. Rings 34 of tubular body 6 are designed to lean against the roller elements placed on the inner surface of the lateral wall of tank 2, and may be shaped as guides, such that their cross-section essentially exhibits a "U" shape, wherein the above-mentioned roller elements are fitted, thus preventing the displacement of tubular body 6 in the longitudinal direction.

In order to prevent unnecessary heat loss in the interior of pressure tank 2, the walls of main portion 8 and of front portion 13 of pressure tank 2, which are made of carbon steel, are coated with a layer of thermal insulation material 28, such as rock wool or similar, coated with an embellishing outer layer 29, preferably made of stainless steel.

The characteristics of the device 1 represented in Figs. 1 to 3 make it particularly suitable for the treatment of urban solid waste 17, which is also an object of the invention.

This method comprises a preliminary stage designed to control and prepare the waste that has been collected and temporarily stored in appropriately prepared facilities. In this preliminary stage, the waste is subject to visual control in order to detect potentially hazardous elements which might affect the safety of the subsequent treatment stages. Preferably, a gauge control is also performed on the waste, in order to prevent the entry of exceedingly voluminous elements into device 1. Optionally, the waste may be subject to a grinding process by a mechanical system.

The main stage in the process takes place in the interior of device 1, and basically consists of the sanitation and drying of the waste 17 obtained during the previous control and preparation stage.

In a first step, waste 17 is introduced, at atmospheric pressure, into tubular body 6 of device 1. In order to achieve this, it is expected to use a telescopic conveyor belt, the free end whereof is introduced into the above-mentioned cylindrical body 6 through the snout of its open end 6c, which transports waste 17 and deposits it inside tubular body 6. Whilst waste 17 is being deposited inside tubular body 6, the latter is made to rotate at constant velocity in order that helicoidal blades 16 may push the waste towards the closed end of tubular body 6.

In order to prevent a potential vertical displacement of the conveyor belt's free end, which might damage tubular body 6, pressure tank 2 may be equipped, in its opening 3, with supports designed to receive the content of the conveyor belt whilst the loading of waste 17 is taking place.

In a subsequent step, pressure tank 2 is closed by means of gate 30, which may be hermetically coupled to opening 3 of the pressure tank, and, whilst tubular body 6 is made to rotate at constant velocity, water vapour 23 begins to be introduced into the pressure tank through inlets 4 and will be appropriately distributed in the interior by means of diffuser elements 12. This water vapour 23, which is introduced at a temperature above 115º and below 200º, will make it possible to achieve the optimal temperature and pressure level inside pressure tank 2 in order to perform sanitation of the waste 17 contained therein.

The low-density plastics which make up most of the wrappers and rubbish bags introduced into pressure tank 2 begin to retract, in such a way that the waste contained therein is released without the need to subject said containers to a previous tearing or grinding process. Furthermore, due to the constant movement whereto waste 17 is subject in the interior of tubular body 6 as a result of the action of longitudinal blades 15, said waste begins to release liquids 25, which flow to the exterior of tubular body 6 through the perforations 7 provided in its lateral wall 6b.

Once the optimal pressure level and temperature are achieved in the interior of pressure tank 2, they are kept constant for between 20 and 90 minutes, depending on the type of waste under treatment, whilst tubular body 6 continues to rotate around its axis. The pressure tank is equipped with a system designed to measure the level of liquids 25 accumulated on the lowermost part 14 thereof, such that, when the level exceeds a certain predetermined limit, they are extracted through outlet 5, which is equipped with a drainage valve. The potential pressure and temperature losses caused by the opening of the drainage valve, as well as by the heat losses through the walls of pressure tank 2, are controlled by different sensors provided on device 1, in a well-known manner, such that said losses are compensated by the introduction of more water vapour 23 into pressure tank 2.

During this stage, formation of the homogeneous mass of waste is completed, whilst the level of humidity inside pressure tank 2 is reduced and practically all the liquids contained in the waste and the condensed vapour are eliminated.

Once the sanitation period is over, the subsequent step of depressurising pressure tank 2 is performed. To this end, the entry of water vapour 23 is completely interrupted without interrupting the rotational movement of tubular body 6. At the same time, the drainage valve is fully opened in order that all the excess liquid accumulated in the lowermost part 14 of pressure tank 2, as well as the existing water vapour 23, may be extracted through outlet 5.

Once the pressure inside pressure tank 2 is practically equal to the atmospheric pressure, hot dry air, or air at ambient temperature, is introduced into the pressure tank through inlets 4. Said air is directed against tubular body 6 through diffuser means 12 and allows for the extraction of the vapour that still remains in the interior of pressure tank 2. Moreover, the same air makes it possible to cool and dry the waste contained in tubular body 6, reducing the level of humidity even further, to less than 15%. In this way, the same pressure tank 2 is first used as a device designed for sanitation of the waste and subsequently as a drying tunnel for the treated waste.

The cooling of the waste described above and the extraction of all the water vapour contained in pressure tank 2 is essential to prevent undesired vapour emissions during the opening of pressure tank 2, as well as during the extraction of the waste and the subsequent separation processes.

In order to extract waste 17, the rotational movement of tubular body 6 is first stopped and, subsequently, gate 30 is removed, in order to access the interior of pressure tank 2. Subsequently, tubular body 6 is made to rotate in the rotational direction opposite that of the loading process of waste 17, causing the movement of waste 17 towards opening 3 as a result of the thrust of helicoidal blades 16.

## Claims

1. Waste treatment method, particularly designed for the treatment of urban solid waste (17), comprising the following steps:
a) introducing the waste to be treated into a tubular body (6) the lateral walls whereof (6b) are equipped with multiple perforations (7);
b) enclosing the tubular body (6) in a sealed working enclosure and injecting a gas therein at a temperature between 115°C and 200°C to subject the assembly formed by the tubular body and the waste introduced therein to a gas working environment at a pressure above 4 bar and a temperature between 115ºC and 200ºC, for at least 15 minutes, and, simultaneously, rotating the tubular body around its longitudinal axis (19), in order to mechanically agitate the introduced waste, the perforations (7) of the lateral walls (6b) being dimensioned to prevent the waste flowing towards the exterior of the tubular body but allowing the liquids (25) released by the waste flowing to the exterior of the tubular body through the perforations in the lateral walls thereof; and
c) gradually reducing the pressure of the gas working environment until it reaches the initial atmospheric pressure through the extraction of the gas from the working enclosure and, simultaneously, cooling and drying the waste (17) introduced into the tubular body (6) through the injection of a drying gas into the working enclosure which reaches the waste through the perforations (7) the lateral walls (6b) of the tubular body (6).

2. Method according to claim 1, **characterised in that**, whilst the simultaneous operations of reducing the pressure of the gas working environment and cooling and drying the waste (17) are being performed, the tubular body (6) rotates around its longitudinal axis (19)

3. Method according to claims 1 or 2, **characterised in that** the liquids (25) which flow to the exterior of the tubular body (6) through the perforations (7) in the lateral walls (6b), as well as the water vapour condensed in the interior of the working enclosure, are collected by a back wall of the above-mentioned working enclosure, and **in that** the method comprises the step of extracting said collected liquids from the working enclosure in a controlled manner, thereby reducing the level of humidity inside said working enclosure, and, consequently, the level of humidity of the waste.

4. Method according to any one of claims 1 to 3, **characterised in that** the gas injected into the working enclosure is directed against the tubular body (6) by means of diffuser devices (12) which are placed facing one another and regularly distributed along the length of the tubular body.

5. Method according to any one of claims 1 to 4, **characterised in that** the drying gas is injected into the working enclosure and directed against the tubular body (6) by means of diffuser devices (12) which are placed facing one another and regularly distributed along the length of the tubular body.

6. Device (1) designed for the treatment of waste (17), particularly for the treatment of urban solid waste, which comprises a pressure tank (2) equipped with at least one opening (3) for the introduction and extraction of the waste therefrom, at least one inlet (4) for a gas, such as water vapour (23) or air, and at least one outlet (5) for the condensates, the liquids (25) which the waste releases during the treatment process and the gas itself, **characterised in that** wherein said pressure tank is fixed and **in that** the device comprises, in addition, a cylindrical tubular body (6), rotable around its longitudinal axis, open on one end (6c), and closed on the opposite end by means of a lid (6a), which is located in the interior of the pressure tank and is designed to contain the waste introduced into the tank and to mechanically agitate it when the tubular body rotates around its longitudinal axis (19), **characterised in that** the lateral walls (6b) of the tubular body are equipped with multiple perforations (7) which are dimensioned to let the gas penetrate into the tubular body, to let the liquids released by the waste flow through said perforations towards the exterior of the tubular body, being collected by the pressure tank, and to prevent the waste flowing towards the exterior of the tubular body, and **in that** the device comprises means for gradually reducing the pressure of the gas working environment until it reaches the initial atmospheric pressure through the extraction of the gas from the working enclosure and, simultaneously, coding and drying the waste (17) introduced into the tubular body (6) through the injection of a drying gas into the working enclosure, which reaches the waste through the perforations (7) in the lateral walls (6b) of the tubular body (6).

7. Device (1) according to claim 6, **characterised in that** the pressure tank (2) comprises a main cylindrical tubular portion (8), closed on the rear end by an outer lid (9), and **in that** the tubular body (6) is placed coaxially with respect to said main portion and, by using sliding means, leans against the inner surface of the pressure tank walls, with an intermediate chamber (10) being formed between the main portion of the pressure tank and the tubular body.

8. Device (1) according to claim 7, **characterised in that** the lid (6a) of the tubular body (6) is placed facing the outer lid (9) of the main portion (8) of the pressure tank (2) and is joined to a driving axis (20) which crosses the above-mentioned outer lid of the main portion of the pressure tank through a sealed joint (22) and is coupled to an engine located on the exterior of said pressure tank.

9. Device (1) according to claims 7 or 8, **characterised in that** the pressure tank (2) comprises a hollow tubular front portion (13) with an increasing cross-section, which is joined without interruption to the open end of the main portion (8) on the end with the largest cross-section, the opening (3) of the pressure tank being located on the end with the smallest cross-section, with a detachable gate (30) that may be hermetically coupled to the above-mentioned opening.

10. Device (1) according to any one of claims 7 to 9, **characterised in that** the tubular body (6) practically covers the entire main portion (8) of the pressure tank (2), and by the fact the open end (6c) of the above-mentioned tubular body protrudes from the snout of the inlet opening (3) of said pressure tank.

11. Device (1) according to any one of claims 7 to 10, **characterised in that** the tubular body (6) is internally equipped with a series of longitudinal blades (15) and/or helicoidal blades (16) which protrude from the inner surface of the lateral wall of the above-mentioned tubular body and are designed to stir and to longitudinally displace, respectively, the waste (17) introduced into said tubular body when the tubular body rotates, in one direction or another, around its longitudinal axis (19).

12. Device (1) according to any one of claims 7 to 11, **characterised in that** the inlets (4) for the gas are placed on the upper part of the lateral wall of the main cylindrical portion (8) of the pressure tank (2).

13. Device (1) according to any one of claims 9 to 12, **characterised in that** the main cylindrical portion (8) of the pressure tank (2) is tilted with respect to the horizontal, in order to allow for the channelling and conduction of the liquid (25) collected by the pressure tank towards the lowermost part (14) of the above-mentioned pressure tank, and **in that** the outlet (5) thereof is placed on this lowermost part, the outlet being equipped with a valve element designed for the controlled extraction of the liquids accumulated in said lowermost part to the exterior of the pressure tank.

14. Device (1) according to any one of claims 6 to 13, **characterised in that** the tubular body (6) comprises a frame, covered by perforated plates which are joined to one another by joining means.

15. Device (1) according to the previous claim, **characterised in that** the frame of the tubular body (6) is composed of rings (34) which are transversely placed with respect to its longitudinal axis (19) and separated by a certain distance, joined to one another by means of longitudinal rods (35).

## Patentansprüche

1. Verfahren zur Behandlung von Abfall, besonders ausgebildet zur Behandlung von Siedlungsabfall (17), umfassend folgende Schritte:
a) Einführen des zu behandelnden Abfalls in einen rohrförmigen Körper (6), wobei die Seitenwände (6b) desselben mit mehreren Löchern (7) versehen sind;
b) Einschließen des rohrförmigen Körpers (6) in einem abgedichteten Arbeitsraum und Einblasen eines Gases in denselben bei einer Temperatur zwischen 115°C und 200°C, um die Baugruppe aus rohrförmigem Körper und in diesem eingeführten Abfall einer Gasarbeitsumgebung bei einem Druck über 4 Bar und einer Temperatur zwischen 115°C und 200°C, während zumindest 15 Minuten, auszusetzen, und gleichzeitig Drehen des rohrförmigen Körpers um seine Längsachse (19), um den eingeführten Abfall mechanisch zu rühren, wobei die Löcher (7) der Seitenwände (6b) so bemessen sind, dass sie den Abfallfluss nach außen in Bezug auf den rohrförmigen Körper verhindern, aber ermöglichen, dass die Flüssigkeit (25), die von dem Abfall befreit wird, nach außen in Bezug auf den rohrförmigen Körper durch die Löcher in den Seitenwänden desselben fließt; und
c) stufenweises Verringern des Drucks der Gasarbeitsumgebung bis er den anfänglichen atmosphärischen Druck durch die Entnahme des Gases aus dem Arbeitsraum erreicht und, gleichzeitig, Abkühlen und Trocknen des in dem rohrförmigen Körper (6) eingeführten Abfalls (17) durch das Einblasen eines Trocknungsgases in den Arbeitsraum, welcher den Abfall durch die Löcher (7) in den Seitenwänden (6b) des rohrförmigen Körpers (6) erreicht.

2. Verfahren nach Anspruch 1, **dadurch gekennzeichnet, dass** während die gleichzeitigen Vorgänge der Verringerung des Drucks der Gasarbeitsumgebung und des Abkühlens und Trocknens des Abfalls (17) durchgeführt werden, der rohrförmige Körper (6) um seine Längsachse (19) rotiert.

3. Verfahren nach den Ansprüchen 1 oder 2, **dadurch gekennzeichnet, dass** die Flüssigkeit (25), welche nach außen in Bezug auf den rohrförmigen Körper (6) durch die Löcher (7) in den Seitenwänden (6b) fließt sowie der Wasserdampf, welcher im Inneren des Arbeitsraums kondensiert, von einer hinteren Wand des oben genannten Arbeitsraums gesammelt wird, und dass das Verfahren den Schritt der Entnahme der genannten gesammelten Flüssigkeit aus dem Arbeitsraum kontrolliert umfasst, wobei auf diese Weise das Feuchtigkeitsniveau innerhalb des genannten Arbeitsraums und, somit, das Feuchtigkeitsniveau des Abfalls verringert wird.

4. Verfahren nach einem der Ansprüche 1 bis 3, **dadurch gekennzeichnet, dass** das in dem Arbeitsraum eingeblasene Gas gegen den rohrförmigen Körper (6) über Diffusionsvorrichtungen (12), welche gegenüberliegend angeordnet und gleichmäßig über die Länge des rohrförmigen Körpers verteilt sind, gerichtet wird.

5. Verfahren nach einem der Ansprüche 1 bis 4, **dadurch gekennzeichnet, dass** das Trocknungsgas in den Arbeitsraum eingeblasen und gegen den rohrförmigen Körper (6) über Diffusionsvorrichtungen (12), welche gegenüberliegend angeordnet und gleichmäßig über die Länge des rohrförmigen Körpers verteilt sind, gerichtet wird.

6. Vorrichtung (1), welche zur Behandlung von Abfall (17), besonders zur Behandlung von Siedlungsabfall, ausgebildet ist, umfassend einen Druckbehälter (2), welcher mit zumindest einer Öffnung (3) für die Einführung und Entnahme des Abfalls aus demselben, zumindest einem Einlass (4) für ein Gas, wie Wasserdampf (23) oder Luft, und zumindest einem Auslass (5) für das Kondensat, die Flüssigkeit (25), welche der Abfall beim Behandlungsprozess befreit und dem Gas selbst, versehen ist, wobei der genannte Druckbehälter fest ist und die Vorrichtung zusätzlich einen zylindrischen rohrförmigen Körper (6) umfasst, welcher um seine Längsachse rotierbar ist, an einem Ende (6c) geöffnet und am entgegengesetzten Ende über einen Deckel (6a) geschlossen ist, wobei sich der Körper (6) im Inneren des Druckbehälters befindet und dazu ausgebildet ist, den in den Behälter eingeführten Abfall zu enthalten und diesen mechanisch zu rühren, wenn der rohrförmige Körper um seine Längsachse (19) rotiert, **dadurch gekennzeichnet, dass** die Seitenwände (6b) des rohrförmigen Körpers mit mehreren Löchern (7) versehen sind, welche so bemessen sind, dass sie den Gasdurchgang in den rohrförmigen Körper zulassen, um zu ermöglichen, dass die Flüssigkeit, die von dem Abfall befreit wird, durch die genannten Löcher nach außen in Bezug auf den rohrförmigen Körper fließt, unter Sammlung in dem Druckbehälter, und um zu verhindern, dass der Abfall nach außen in Bezug auf den rohrförmigen Körper fließt, und dass die Vorrichtung Mittel zum stufenweisen Verringern des Drucks der Gasarbeitsumgebung bis er den anfänglichen atmosphärischen Druck durch die Entnahme des Gases aus dem Arbeitsraum erreicht und, gleichzeitig, zum Abkühlen und Trocknen des in dem rohrförmigen Körper (6) eingeführten Abfalls (17) durch das Einblasen eines Trocknungsgases in den Arbeitsraum, welcher den Abfall durch die Löcher (7) in den Seitenwänden (6b) des rohrförmigen Körpers (6) erreicht, umfasst.

7. Vorrichtung (1) nach Anspruch 6, **dadurch gekennzeichnet, dass** der Druckbehälter (2) einen zylindrischen und rohrförmigen Hauptbereich (8) umfasst, welcher an dem hinteren Ende von einem äußeren Deckel (9) geschlossen ist und dass der rohrförmige Körper (6) in Bezug auf den genannten Hauptbereich koaxial angeordnet ist und, durch die Verwendung von Gleitmitteln, lehnt er sich gegen der inneren Oberfläche der Druckbehälterwände, wobei eine Zwischenkammer (10) zwischen dem Hauptbereich des Druckbehälters und dem rohrförmigen Körper gebildet ist.

8. Vorrichtung (1) nach Anspruch 7, **dadurch gekennzeichnet, dass** der Deckel (6a) des rohrförmigen Körpers (6) dem äußeren Deckel (9) des Hauptbereichs (8) des Druckbehälters (2) gegenüberliegend angeordnet ist und mit einer Antriebsachse (20) verbunden ist, welche den oben genannten äußeren Deckel des Hauptbereichs des Druckbehälters durch eine Dichtung (22) kreuzt und mit einem Motor, welcher sich außerhalb des genannten Druckbehälters befindet, gekoppelt ist.

9. Vorrichtung (1) nach den Ansprüchen 7 oder 8, **dadurch gekennzeichnet, dass** der Druckbehälter (2) einen hohlen rohrförmigen Vorderbereich (13) mit einem zunehmenden Querschnitt umfasst, welcher ohne Durchbrechung mit dem offenen Ende des Hauptbereichs (8) an dem Ende mit dem größten Querschnitt verbunden ist, wobei sich die Öffnung (3) des Druckbehälters an dem Ende mit dem kleinsten Querschnitt befindet, mit einer abtrennbaren Klappe (30), die mit der oben genannten Öffnung hermetisch koppelbar ist.

10. Vorrichtung (1) nach einem der Ansprüche 7 bis 9, **dadurch gekennzeichnet, dass** der rohrförmige Körper (6) praktisch den ganzen Hauptbereich (8) des Druckbehälters (2) abdeckt und durch die Tatsache, dass das offene Ende (6c) des oben genannten rohrförmigen Körpers von der Mündung der Einlassöffnung (3) des genannten Druckbehälters hinausragt.

11. Vorrichtung (1) nach einem der Ansprüche 7 bis 10, **dadurch gekennzeichnet, dass** der rohrförmige Körper (6) im Inneren mit mehreren longitudinalen Flügeln (15) und/oder spindelförmigen Flügeln (16) versehen ist, welche von der inneren Oberfläche der Seitenwand des oben genannten rohrförmigen Körpers hinausragen und dazu ausgebildet sind, den in dem genannten rohrförmigen Körper eingeführten Abfall (17) jeweils zu schütteln und longitudinal zu bewegen, wenn der rohrförmige Körper rotiert, in eine oder in die andere Richtung, um seine Längsachse (19).

12. Vorrichtung (1) nach einem der Ansprüche 7 bis 11, **dadurch gekennzeichnet, dass** die Einlässe (4) für das Gas auf dem oberen Teil der Seitenwand des zylindrischen Hauptbereichs (8) des Druckbehälters (2) angeordnet sind.

13. Vorrichtung (1) nach einem der Ansprüche 9 bis 12, **dadurch gekennzeichnet, dass** der zylindrische Hauptbereich (8) des Druckbehälters (2) in Bezug auf die Horizontale geneigt ist, um die Leitung und Führung der Flüssigkeit (25), welche von dem Druckbehälter gesammelt wird, zum untersten Teil (14) des oben genannten Druckbehälters zu ermöglichen, und dass sein Auslass (5) auf diesem untersten Teil angeordnet ist, wobei der Auslass mit einem Ventilelement versehen ist, welches für die kontrollierte Entnahme der in dem genannten untersten Teil gesammelten Flüssigkeit zum Äußeren des Druckbehälters ausgebildet ist.

14. Vorrichtung (1) nach einem der Ansprüche 6 bis 13, **dadurch gekennzeichnet, dass** der rohrförmige Körper (6) einen Rahmen umfasst, welcher von gelochten Platten, die miteinander über Verbindungsmittel verbunden sind, bedeckt ist.

15. Vorrichtung (1) nach dem vorhergehenden Anspruch, **dadurch gekennzeichnet, dass** der Rahmen des rohrförmigen Körpers (6) aus Ringen (34) besteht, welche in Bezug auf seine Längsachse (19) transversal angeordnet und durch einen bestimmten Abstand getrennt sind, und miteinander über longitudinale Stäbe (35) verbunden sind.

## Revendications

1. Procédé pour le traitement de déchets, particulièrement conçu pour le traitement de déchets solides urbains (17) comprenant les étapes suivantes :
a) introduire les déchets à traiter dans un corps tubulaire (6) dont les parois latérales (6b) sont équipées de multiples perforations (7) ;
b) enfermer le corps tubulaire (6) dans une enceinte de travail étanche et injecter un gaz en son sein à une température entre 115°C et 200°C pour soumettre l'ensemble formé par le corps tubulaire et les déchets introduits en son sein à un environnement de travail du gaz à une pression supérieure à 4 bar et une température entre 115°C et 200°C, pendant au moins 15 minutes, et, simultanément, faire tourner le corps tubulaire autour de son axe longitudinal (19) afin d'agiter mécaniquement les déchets introduits, les perforation (7) des parois latérales (6b) étant dimensionnées pour éviter que les déchets s'écoulent vers l'extérieur du corps tubulaire mais permettre que les liquides (25) libérés par les déchets s'écoulent vers l'extérieur du corps tubulaire à travers les perforations dans les parois latérales de celui-ci ; et
c) réduire graduellement la pression de l'environnement de travail du gaz jusqu'à atteindre la pression atmosphérique initiale à travers l'extraction du gaz de l'enceinte de travail et simultanément, refroidir et sécher les déchets (17) introduits dans le corps tubulaire (6) à travers l'injection d'un gaz de séchage dans l'enceinte de travail, qui atteint les déchets à travers les perforations (7) dans les parois latérales (6b) du corps tubulaire (6).

2. Procédé selon la revendication 1, **caractérisé en ce que**, pendant que les opérations simultanées de réduction de la pression de l'environnement de travail du gaz et de refroidissement et séchage des déchets (17) sont réalisées, le corps tubulaire (6) tourne autour de son axe longitudinal (19).

3. Procédé selon les revendications 1 ou 2, **caractérisé en ce que** les liquides (25) qui s'écoulent vers l'extérieur du corps tubulaire (6) à travers les perforations (7) dans les parois latérales (6b), ainsi que la vapeur d'eau condensée à l'intérieur de l'enceinte de travail, sont collectés par une paroi arrière de ladite enceinte de travail, et **en ce que** le procédé comprend l'étape d'extraire lesdits liquides collectés de l'enceinte de travail d'une manière contrôlée, en réduisant de la sorte le niveau d'humidité à l'intérieur de ladite enceinte de travail, et par conséquent, le niveau d'humidité des déchets.

4. Procédé selon l'une quelconque des revendications 1 à 3, **caractérisé en ce que** le gaz injecté dans l'enceinte de travail est dirigé contre le corps tubulaire (6) par le biais de dispositifs diffuseurs (12) qui sont mis en place les uns en regard des autres et distribués régulièrement le long de la longueur du corps tubulaire.

5. Procédé selon l'une quelconque des revendications 1 à 4, **caractérisé en ce que** le gaz de séchage est injecté dans l'enceinte de travail et dirigé contre le corps tubulaire (6) par le biais de dispositifs diffuseurs (12) qui sont mis en place les uns en regard des autres et distribués régulièrement le long de la trajectoire du corps tubulaire.

6. Dispositif (1) conçu pour le traitement de déchets (17), en particulier pour le traitement de déchets solides urbains, qui comprend un réservoir de pression (2) équipé d'au moins une ouverture (3) pour l'introduction et l'extraction des déchets de celui-ci, au moins une entrée (4) pour un gaz, tel que de la vapeur d'eau (23) ou de l'air, et au moins une sortie (5) pour les condensats, les liquides (25) que les déchets libèrent pendant le procédé de traitement et le propre gaz, dans lequel ledit réservoir de pression est fixé et en ce que le dispositif comprend, en outre, un corps tubulaire cylindrique (6), rotatif autour de son axe longitudinal, ouvert sur une extrémité (6c), et fermé sur l'extrémité opposée par le biais d'un couvercle (6a), qui est situé à l'intérieur du réservoir de pression et est conçu pour contenir les déchets introduits dans le réservoir et pour les agiter mécaniquement lorsque le corps tubulaire tourne autour de son axe longitudinal (19), **caractérisé en ce que** les parois latérales (6b) du corps tubulaire sont équipées de multiples perforations (7) qui sont dimensionnées pour laisser pénétrer le gaz dans le corps tubulaire, pour laisser que les liquides libérés par les déchets s'écoulent à travers lesdites perforations vers l'extérieur du corps tubulaire, en étant collectés par le réservoir de pression, et pour éviter que les déchets s'écoulent vers l'extérieur du corps tubulaire, et **en ce que** le dispositif comprend des moyens pour réduire graduellement la pression de l'environnement de travail du gaz jusqu'à atteindre la pression atmosphérique initiale à travers l'extraction du gaz de l'enceinte de travail et simultanément refroidir et sécher les déchets (17) introduits dans le corps tubulaire (6) à travers l'injection d'un gaz de séchage dans l'enceinte de travail, qui atteint les déchets à travers les perforations (7) dans les parois latérales (6b) du corps tubulaire (6).

7. Dispositif (1) selon la revendication 6, **caractérisé en ce que** le réservoir de pression (2) comprend une portion tubulaire cylindrique principale (8), fermé sur l'extrémité arrière par un couvercle extérieur (9), et **en ce que** le corps tubulaire (6) est mis en place de manière coaxiale par rapport à ladite portion principale et, en utilisant des moyens coulissants, s'appuie contre la surface intérieure des parois du réservoir de pression, une chambre intermédiaire (10) étant formée entrer la portion principale du réservoir de pression et le corps tubulaire.

8. Dispositif (1) selon la revendication 7, **caractérisé en ce que** le couvercle (6a) du corps tubulaire (6) est mis en place en regard du couvercle extérieur (9) de la portion principale (8) du réservoir de pression (2) et est uni à un axe moteur (20) qui traverse ledit couvercle extérieur de la portion principale du réservoir de pression à travers un joint d'étanchéité (22) et est accouplé à un moteur situé à l'extérieur dudit réservoir de pression.

9. Dispositif (1) selon les revendications 7 ou 8, **caractérisé en ce que** le réservoir de pression (2) comprend une portion avant tubulaire creuse (13) avec une section transversale croissante, qui est unie sans interruption à l'extrémité ouverte de la portion principale (8) sur l'extrémité ayant la section transversale la plus grande, l'ouverture (3) du réservoir de pression étant située sur l'extrémité ayant la section transversal la plus petite, avec une porte détachable (30) qui peut être accouplé hermétiquement à ladite ouverture.

10. Dispositif (1) selon l'une quelconque des revendications 7 à 9, **caractérisé en ce que** le corps tubulaire (6) recouvre pratiquement toute la portion principale (8) du réservoir de pression (2), et par le fait que l'extrémité ouverte (6c) dudit corps tubulaire dépasse de la pointe de l'ouverture d'entrée (3) dudit réservoir de pression.

11. Dispositif (1) selon l'une quelconque des revendications 7 à 10, **caractérisé en ce que** le corps tubulaire (6) est équipé intérieurement d'une série de lames longitudinales (15) et/ou de lames hélicoïdales (16) qui dépassent de la surface intérieure de la paroi latérale dudit corps tubulaire et sont conçues pour agiter et pour déplacer longitudinalement, respectivement, les déchets (17) introduits dans ledit corps tubulaire lorsque le corps tubulaire tourne, dans une direction ou une autre, autour de son axe longitudinal (19).

12. Dispositif (1) selon l'une quelconque des revendications 7 à 11, **caractérisé en ce que** les entrées (4) pour le gaz sont mises en place sur la partie supérieure de la paroi latérale de la portion cylindrique principal (8) du réservoir de pression (2).

13. Dispositif (1) selon l'une quelconque des revendications 9 à 12, **caractérisé en ce que** la portion cylindrique principale (8) du réservoir de pression (2) est inclinée par rapport à l'horizontale, afin de permettre la canalisation et la conduction du liquide (25) collecté par le réservoir de pression vers la partie plus inférieure (14) dudit réservoir de pression, et **en ce que** sa sortie (5) est mise en place sur la partie plus inférieure, la sortie étant équipée d'un élément de soupape conçu pour l'extraction contrôlée des liquides accumulés dans ladite partie plus inférieur inférieure vers l'extérieur du réservoir de pression.

14. Dispositif (1) selon l'une quelconque des revendications 6 à 13, **caractérisé en ce que** le corps tubulaire (6) comprend un châssis, recouvert de plaques perforées qui sont unies les unes aux autres par des moyens d'union.

15. Dispositif (1) selon la revendication précédente, **caractérisé en ce que** le châssis du corps tubulaire (6) est composé d'anneaux (34) qui sont mis en place transversalement par rapport à son axe longitudinal (19) et séparés par une certaine distance, unis les uns aux autres par le biais de tringles longitudinales (35).
